# EUROPEAN PATENT SPECIFICATION

(11) **EP 3 451 932 B1**
(45) Date of publication and mention of the grant of the patent: **28.10.2020**
(21) Application number: 17726370.4
(22) Date of filing: 03.05.2017
(51) Int. Cl.: A61B 8/00, A61B 8/08

(54) **ULTRASONIC IMAGING SYSTEM WITH SIMPLIFIED 3D IMAGING CONTROLS**
ULTRASCHALLBILDGEBUNGSSYSTEM MIT VEREINFACHTEN 3D-BILDGEBUNGSSTEUERUNGEN
SYSTÈME D'IMAGERIE PAR ULTRASONS DOTÉ DE COMMANDES D'IMAGERIE 3D SIMPLIFIÉES

(30) Priority: 06.05.2016 US 201662332687 P
(43) Date of publication of application: 13.03.2019
(73) Proprietor: Koninklijke Philips N.V., 5656 AG Eindhoven (NL)
(72) Inventor: AGARWAL, Anup, 5656 AE Eindhoven (NL); JAGO, James Robertson, 5656 AE Eindhoven (NL); ENTREKIN, Robert Randall, 5656 AE Eindhoven (NL); BROWN, Jimmy Ray, 5656 AE Eindhoven (NL); BANNISTER, Barbara, 5656 AE Eindhoven (NL)
(74) Representative: Philips Intellectual Property & Standards
(86) International application number: PCT/IB2017/052559
(87) International publication number: WO 2017/191568

(56) References cited:
- EP-A2- 2 733 947
- US-A1- 2009 099 449
- US-A1- 2009 304 250

## Description

This invention relates to medical ultrasound systems and, in particular, to ultrasound systems which perform two-dimensional (2D) and three-dimensional (3D) imaging.

Ultrasound probes are used to transmit ultrasound waves into the body as well as receive the reflected waves. The reflected echoes are transferred to the ultrasound system for additional signal processing and final generation of an image that is displayed on the screen. With recent advances in technology, compact, high density electronics can be fit inside modern-day transducer probes that allows much of the transmit/receiver signal processing to be performed inside the probe itself. This has led to the development of matrix probes that can operate thousands of transducer elements of an ultrasound array that opens up the possibility of new probe geometries and imaging modes. One of the interesting new modes is 3D/4D (live 3D) imaging in B-mode as well as color flow imaging.

For the last thirty years, two-dimensional planar imaging has been the conventional way to view and diagnose pathology and the standard for live ultrasound imaging. Three-dimensional imaging provides the ability to visualize anatomy in three dimensions, enabling clinicians to understand details of pathology from a perspective not previously possible with ultrasound. But 3D imaging also presents new challenges in image acquisition. While 3D has gained rapid adoption in specific scenarios such as rendering baby faces in fetal exams, it has not gained widespread acceptance in general abdominal and vascular imaging. Part of the challenge is the relative unfamiliarity of sonographers with manipulating the system to acquire views of the desired 3D/4D slices and planes. While 3D ultrasound imaging is a very powerful tool, it remains under-utilized primarily due to two reasons. First, clinicians are frequently unfamiliar with the appearance of anatomy in 3D and its use in ultrasound diagnosis. Secondly, many of the system controls for 3D imaging and their interplay are complicated to use. Accordingly, it is desirable to simplify the controls for 3D ultrasound imaging so that the images needed for a diagnosis can be readily obtained by those who are unfamiliar with the use of 3D ultrasound.

EP 2 733 947 A2 discloses a 3D ultrasound image generating apparatus that includes a volume-data generator adapted to generate 3D volume data based on one or more cross-sectional images with respect to a body tissue of a subject, and a controller adapted to generate a final 3D image having an adjusted 3D effect by means of volume rendering the 3D volume data based on an input stereo-depth value.

It is an object of the present invention to provide a 3D ultrasound system which is simple to operate and control, preferably through automation of system setup and controls.

It is a further object of the present invention to simplify the manipulation of controls needed to acquire diagnostically useful images in the 3D mode.

These objects are solved by the independent claim. Advantageous embodiments are defined in the dependent claims.

In one aspect, the present invention includes an ultrasound system for an exam using 3D imaging as defined in claim 1. The ultrasound system includes a transducer array configured to transmit ultrasound waves to a target anatomy and receive ultrasonic echoes in response. The system can also include a processor configured to, based on the received ultrasonic echoes, produce a 2D ultrasound image including the target anatomy, and based on a system input, select a plurality of graphical icons stored in memory on the system, wherein each graphical icon comprises a different 3D thumbnail view of the target anatomy. The system further includes a display that can display the graphical icons and other user interface features.

In some aspects, an ultrasound system is provided that, based on a system input, can provide a user with graphical icons showing 3D thumbnail views of a particular anatomy of interest. In one example, a user input can be used to identify the particular anatomy being viewed in the 2D ultrasound image, and thereby cause the system to generate graphical icons representing different 3D views of the anatomy. In another example, a reference 2D image can be acquired by the system and used directly by the system or a model can be applied to the reference 2D image to identify the anatomy being imaged. With the reference 2D image or model data identifying the orientation of the probe relative to target anatomy, an automated process invokes 3D graphical icons showing views appropriate for the intended exam. In an implementation of an ultrasound system of the present invention described below, the automated process comprises selection and execution of macro instructions which invoke appropriate 3D system imaging and control setups associated with the particular 3D icons showing the 3D views.

In the drawings:
FIGURE 1 illustrates a two dimensional image of target anatomy which is used as a reference image to set up the 3D controls of an ultrasound system in accordance with the principles of the present invention.
FIGURE 2 illustrates an ultrasound system 2D imaging control panel with a quick-launch key to switch to a 3D imaging mode.
FIGURE 3 illustrates a simplified 3D imaging control panel which is set up in accordance with the present invention.
FIGURE 4 illustrates the control panel of FIGURE 3 when partially set up for a carotid exam in accordance with the present invention.
FIGURE 5 illustrates the control panel of FIGURE 3 when fully set up for a carotid exam in B mode in accordance with the present invention.
FIGURE 6 illustrates a number of 3D view options of the carotid which are based upon a given reference image.
FIGURE 7 illustrates the control panel of FIGURE 5 when switched to the color flow mode for a 3D exam of the carotid in accordance with the present invention.
FIGURE 8 illustrates the control panel of FIGURE 5 when switched to the vessel cast (power Doppler) mode for a 3D exam of the carotid in accordance with the present invention.
FIGURE 9 illustrates in block diagram form a 2D/3D ultrasound system constructed in accordance with the principles of the present invention.

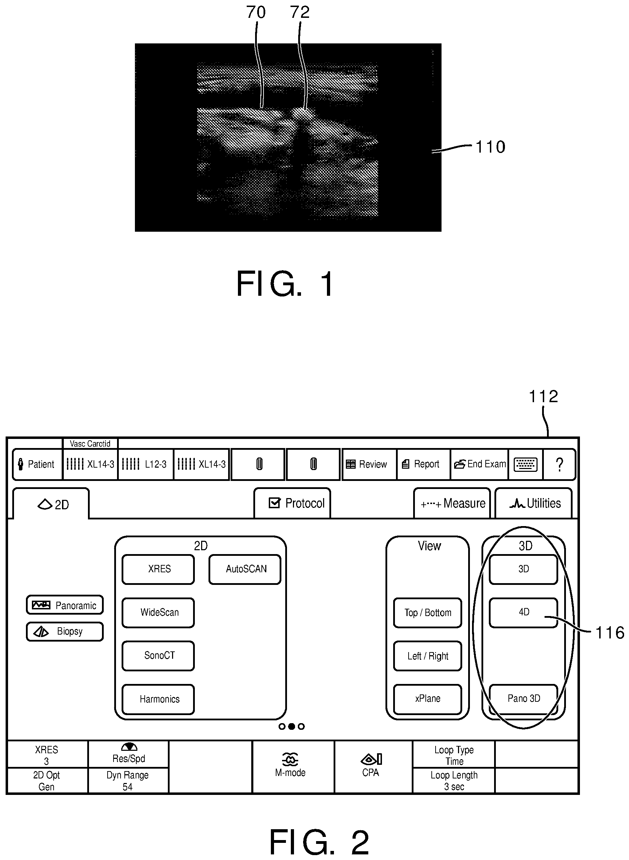

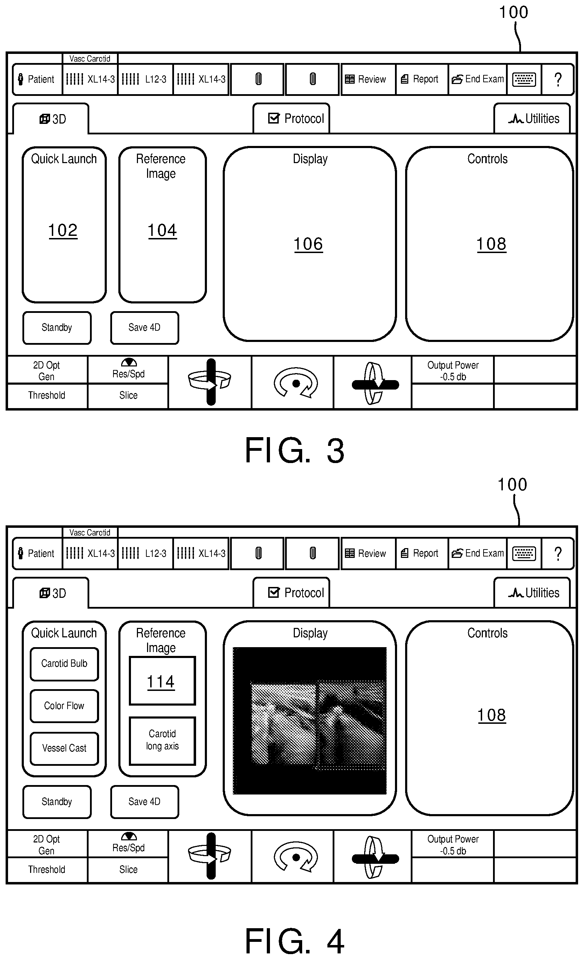

Referring first to FIGURE 1, a 2D ultrasound image 110 of a carotid artery is shown. This image is a typical B mode image of the carotid bulb in a long-axis view as may be acquired during an examination of the carotid artery for signs of plaque. In the image 110 the lumen 70 of the vessel appears black in the image, since this is the way blood flow appears in a B mode image. Also shown in this image is a deposit 72 of plaque in the artery. A clinician seeing this 2D image may want to know more about the plaque deposit 72, such as its shape, surface texture, and extent along or across the vessel, information which is not revealed by a long-axis 2D view. This information may be gleaned from a 3D image of the carotid, which is facilitated in an ultrasound system of the present invention by actuating a quick-launch key to switch to the 3D/4D mode. FIGURE 2 shows a touchscreen user interface 112 for the 2D mode, which a clinician has used to acquire the 2D image of FIGURE 1. At the right side of this 2D user interface are several 3D quick-launch keys indicated by the oval. In this example the clinician actuates the 4D quick-launch key 116, which switches the user interface to the 3D user interface shown in FIGURE 3. The clinician has thereby switched the ultrasound system to the 3D mode with a single click of a control key.

As described herein, the present invention improves 3D workflows for ultrasound users by, for example, providing graphical icons corresponding to different 3D views of a target anatomy. The graphical icons are generated depending, for example, on the specific target anatomy being imaged and/or on the position and orientation of a transducer array in relation to the target anatomy. Different regions of anatomy have different standard views upon which a clinician will rely when making a diagnosis. For instance, if a carotid is being imaged in 2D, then upon activation of 3D imaging a select set of graphical icons showing 3D thumbnails of the target anatomy will appear for the user to choose from in order to more easily generate a 3D ultrasound image of the target anatomy. Similarly, if the user indicates the orientation of the carotid or the system identifies the orientation of the carotid (e.g., via segmentation modeling), then upon activation of 3D imaging a select set of graphical icons showing 3D thumbnails of the target anatomy will appear for the user to choose from in order to more easily generate a 3D ultrasound image.

In certain aspects, the graphical icons will be generated based on a system input that indicates which target anatomy is being imaged and/or position and orientation information regarding the transducer array in relation to the target anatomy. In some instances, the system input can include a user input that indicates orientation information of the target anatomy with respect to the transducer array. For example, a button indicating a specific target anatomy (e.g., a carotid) and/or orientation (e.g., long axis) can be selected by the user. Alternatively, the system input can include a text-based or touch-based input configured to identify a viewing orientation of the target anatomy or protocol configured to view the target anatomy.

In some aspects, the system input can be generated by a 2D ultrasound image in which the system uses the 2D ultrasound as a reference image. The reference image, for example, can be a long axis view of a carotid or a short axis view of the carotid as acquired in a 2D ultrasound image by the ultrasound system. The system can, for example, automatically identify the orientation of the target anatomy in the ultrasound image, or the system can be configured to display the different reference images for user selection. A user input can then include selection of a reference image (e.g., a long axis ultrasound image or a short axis ultrasound image) displayed on the display, and the reference image indicates orientation information of the target anatomy with respect to the transducer array.

FIGURE 3 illustrates one layout of a 3D user interface 100 for an ultrasound system of the present invention. This panel has four areas for 3D/4D imaging. The first area 102 is the "Quick Launch" area 102 which allows a user to launch specific display modes, such as B mode, color flow and color flow Doppler modes. The second area 104, the "Reference Image" area, is an area in which the user is able the specify the probe orientation relative to the anatomy of interest. By defining the probe orientation to the ultrasound system, the system is then able to know the anatomy being imaged and set up the 3D image views most beneficial for a user who is diagnosing that anatomy. Two typical transducer orientations are a long-axis view and a short-axis view, for instance. The third area 106 is the "Display" area. For each quick-launch key and transducer orientation a number of different display options will be available. Each display option is characterized by one or more specific display parameters. Such parameters may include the number of MPRs (multiplanar reformatted slice images) and an associated volume display; ROI (region of interest) box size and location for each MPR image; volume image look direction, and rotation values for A, B, and C planes relative to a reference axis or plane. These display options may be shown in the area 106 in text, but preferably they are shown as thumbnails of the available images as illustrated below. The fourth area 108 is the "Controls" area which presents a list of 3D controls available to the user for each "Display" option.

FIGURE 4 illustrates the 3D touchscreen user interface of FIGURE 3 when the carotid bulb image of FIGURE 1 is specified to the system as the reference image for 3D setup. Specifying a reference image informs the system as to the orientation of the ultrasound probe relative to a target anatomy, in this case, the carotid artery. When the user clicks on the 2D image of the carotid bulb image 110 of FIGURE 1 and then clicks in the "Reference Image" area 114, that image is identified to the ultrasound system as the reference image and a thumbnail of the image appear in area 114 of the user interface. In this example the user has further specified the orientation of the probe relative to the carotid bulb to the system by typing "Carotid" and "long axis" in the entry box that pops up below the defined reference image 114. In a given ultrasound system these manual user selection and entry steps may be performed automatically. For instance, once the 3D user interface 100 is launched, a thumbnail of the ultrasound image in the active image display area, the screen which is displaying carotid bulb image 110 in this example, may automatically be caused to appear as the reference image thumbnail in area 114. If the user had set up the ultrasound system previously for the conduct of a specific exam type in order to acquire the reference image, as by initiating a carotid exam protocol on the system, for instance, the system would know to launch 3D imaging setup for a carotid exam. Features which automatically recognize probe orientation or the anatomical view of an image may be used to automatically enter orientation information in the Reference Image area 104. For instance, an EM (electromagnetic) tracking feature such as the Percunav™ option available for Philips Healthcare ultrasound systems, which automatically follows the orientation of a probe relative to a subject, can be accessed to automatically enter probe orientation information. Image recognition processors such as the "Heart Model" feature available for Philips Healthcare ultrasound systems can also provide orientation information from image recognition processing. See, for instance, the image recognition processors and functions described in US pat. pub. no. 2013/0231564 (Zagorchev et al.) and in US pat. pub. no. 2015/0011886 (Radulescu et al.) With the ultrasound system now knowing that the probe is visualizing a carotid bulb in a long-axis view, one or more display options are presented in the "Display" area. In this example the Display area is showing, on the left, a thumbnail of a 3D volume image acquired by the probe, and, on the right, a thumbnail of an MPR slice image of the carotid artery showing the plaque deposit which has been reconstructed from a 3D data set acquired by the probe.

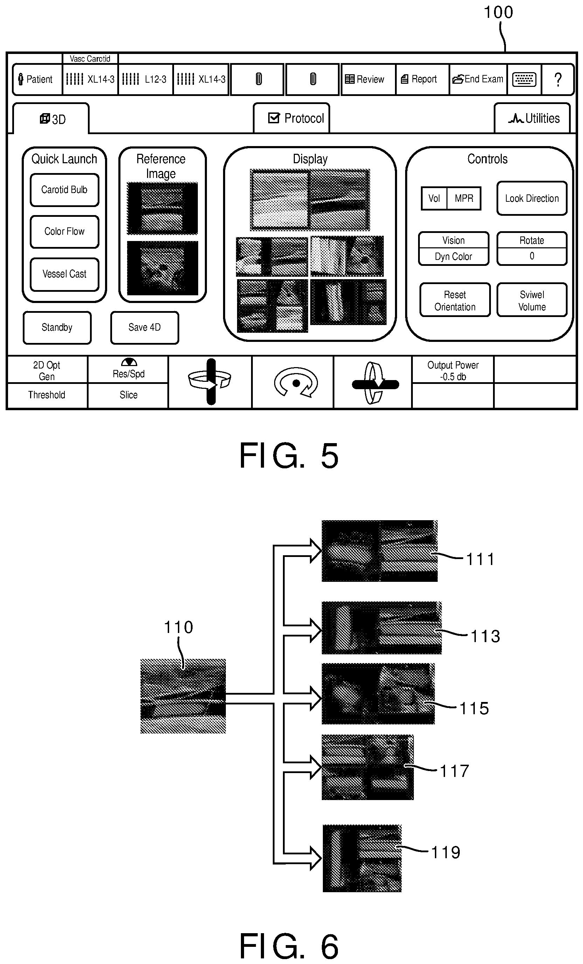

FIGURE 5 illustrates another example of a quick-launched 3D user interface 100 for a carotid artery reference image. In this example two reference images have been specified to the system, the top thumbnail being a long-axis view of the carotid and the bottom thumbnail being a short-axis view of the carotid in an image plane normal to that of the long-axis view. The Display area in this example presents five display options: the top thumbnails are for a 3D volume image and an MPR slice of the long-axis view; the middle-left thumbnails are short-axis volume and long-axis MPR images; the middle-right thumbnails are a rotated long-axis volume view and a short-axis MPR image; the lower-left thumbnails are a long-axis 3D volume image and three orthogonal MPR slice views; and the lower-right thumbnails are a rotated 3D volume image and long-axis and short-axis MPR slices. The "Controls" area 108 is populated with a number of image generation and manipulation controls available to the user when working with any one of the display options, including volume and MPR controls, look direction selection, color control, and a volume rotation control. When the user clicks on one of the thumbnail Display options, the active image display screen of the ultrasound system starts displaying live images in accordance with the selected display option, and with the necessary imaging controls of the Control area activated. As illustrated in FIGURE 6, an identified 2D reference image 110 can thus result in live 3D imaging in any of the available 3D imaging options 111-119.

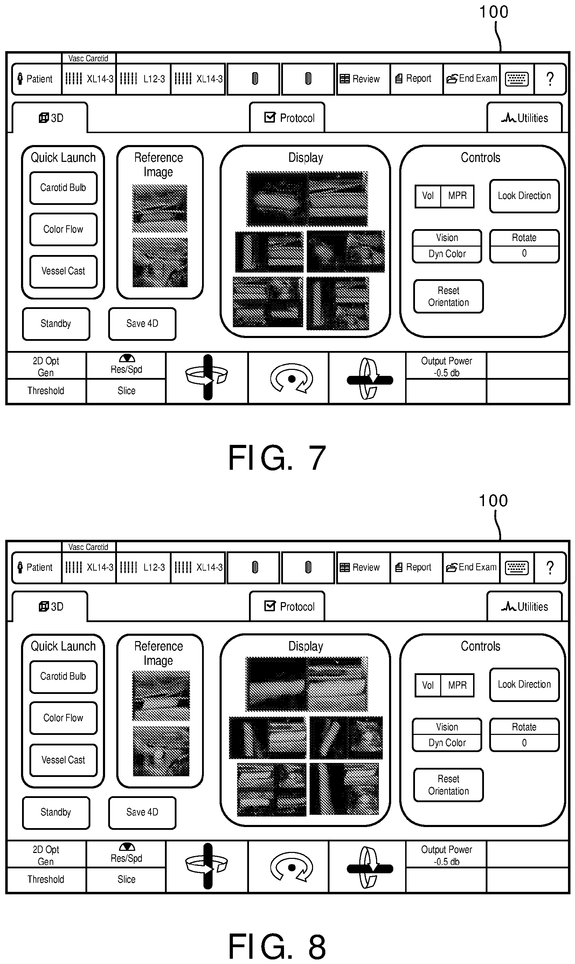

As the user interface 100 of FIGURE 5 illustrates, the "Quick Launch" area 102 may also enable the quick launch of 3D imaging in a specific imaging mode. The example user interface of FIGURE 5 provides the user with mode choices of color flow and vessel case (color power Doppler), in addition to the base B mode. When the user clicks on the "Color Flow" key in the user interface 100 of FIGURE 5, the ultrasound system switches the imaging of the reference image(s) and the display options to the color flow mode as shown in FIGURE 7. As this illustration shows, the lumen of the carotid artery in the images is now filled with color depicting the presence and direction of blood flow in the artery. Again, the user may perform detailed 3D imaging with any of the Display options shown in the center panel of the touchscreen user interface 100, now in the color flow mode. The other mode option which is available in the example of FIGURE 5 is the "Vessel Cast" (color power Doppler) imaging mode. When the user clicks on this key, the images switch to display in the power Doppler mode as illustrated by the thumbnail images in the user interface 100 of FIGURE 8. Other mode selection options may also be made available to a user in a particular implementation of the present invention.

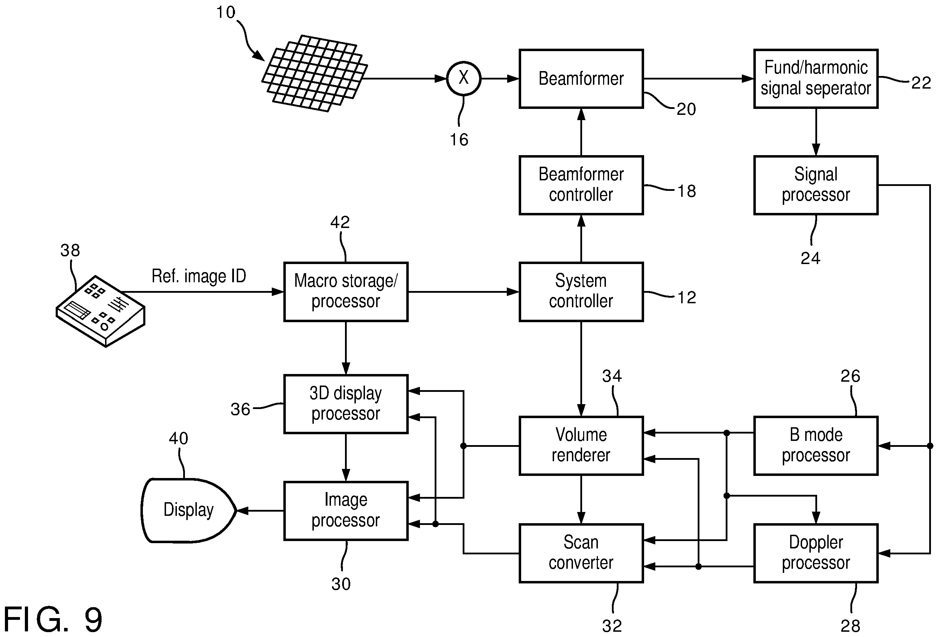

Referring to FIGURE 9, an ultrasound system constructed in accordance with the principles of the present invention for ease in 3D imaging is shown in block diagram form. A two-dimensional transducer array 10 is provided for transmitting ultrasonic waves for imaging and receiving echo signals for image formation. The array is located in an ultrasound probe and is typically mounted in the probe with an integral microbeamformer which controls the transmission of beams in two or three dimensions and the partial beamforming of received echo signals. The array and its microbeamformer are coupled to the mainframe ultrasound system by a transmit/receive (T/R) switch 16 which switches between transmission and reception and protects the receive channels of the system beamformer 20 from high energy transmit signals. The transmission of ultrasonic energy from the transducer array 10 and the formation of coherent echo signals by the microbeamformer and the system beamformer 20 are controlled by a transmit controller 18 coupled to the beamformer 20, which receives input from the user's operation of the user interface or control panel 38, such as selection of a particular imaging mode, from a system controller 12. The echo signals received by elements of the array 10 are partially beamformed by the probe microbeamformer, and the resultant partial sum signals are coupled to the system beamformer 20 where the beamforming process is completed to form coherent beamformed signals.

The beamformed receive signals are coupled to a fundamental/harmonic signal separator 22. The separator 22 acts to separate linear and nonlinear signals so as to enable the identification of the strongly nonlinear echo signals returned from microbubbles or tissue and fundamental frequency signals, both for image formation. The separator 22 may operate in a variety of ways such as by bandpass filtering the received signals in fundamental frequency and harmonic frequency bands (including super-, sub-, and/or ultra-harmonic signal bands), or by a process for fundamental frequency cancellation such as pulse inversion or amplitude modulated harmonic separation. Other pulse sequences with various amplitudes and pulse lengths may also be used for both linear signal separation and nonlinear signal enhancement. A suitable fundamental/harmonic signal separator is shown and described in international patent publication WO 2005/074805 (Bruce et al.) The separated fundamental and/or nonlinear (harmonic) signals are coupled to a signal processor 24 where they may undergo additional enhancement such as speckle removal, signal compounding, and noise elimination.

The processed signals are coupled to a B mode processor 26 and a Doppler processor 28. The B mode processor 26 employs amplitude detection for the imaging of structures in the body such as muscle, tissue, and the walls of blood vessels. B mode images of structures of the body may be formed in either the harmonic mode or the fundamental mode. Tissues in the body and microbubbles both return both types of signals and the stronger harmonic returns of microbubbles enable microbubbles to be clearly segmented in an image in most applications. The Doppler processor 28 processes temporally distinct signals from tissue and blood flow by fast Fourier transformation (FFT) or other Doppler detection techniques for the detection of motion of substances in the image field including blood cells and microbubbles. The Doppler processor may also include a wall filter to eliminate unwanted strong signal returns from tissue in the vicinity of flow such as vessel walls. The anatomic and Doppler flow signals produced by these processors are coupled to a scan converter 32 and a volume renderer 34, which produce image data of tissue structure, flow, or a combined image of both of these characteristics such as a color flow or power Doppler image. The scan converter converts echo signals with polar coordinates into image signals of the desired image format such as a sector image in Cartesian coordinates. The volume renderer 34 converts a 3D data set into a projected 3D image as viewed from a given reference point (look direction) as described in US Pat. 6,530,885 (Entrekin et al.) As described therein, when the reference point of the rendering is changed the 3D image can appear to rotate in what is known as kinetic parallax. Also described in the Entrekin et al. patent is the representation of a 3D volume by planar images of different image planes reconstructed from a 3D image data set, a technique known as multiplanar reformatting. The volume renderer 34 can operate on image data in either rectilinear or polar coordinates as described in US Pat. 6,723,050 (Dow et al.) The 2D or 3D images are coupled from the scan converter and volume renderer to an image processor 30 for further enhancement, buffering and temporary storage for display on an image display 40.

In accordance with the principles of the present invention, the user interface 38, can be embodied, e.g., in both hard key and touch panel form, such as the touchpanel user interfaces 100 and 112 shown and described above, includes a user control by which the system user can identify one or more characteristics of a reference image, such as probe orientation, to the ultrasound system. An example of this was described in conjunction with FIGURES 3 and 4. Alternatively, the target anatomy and/or position and orientation information can be provided by the user, as described above.

Depending on the system input (e.g., the user touching a button or identification of a reference image, a system controller configured to control the ultrasound system based on the system input can be coupled to a macro storage/processor 42. The macro storage/processor stores a number of macro instructions for image acquisition, formation and manipulation which are selected and arranged by the processor in a given number and sequence of macros instructions. The formulated sequence of macros is forwarded to the system controller, which sets up the ultrasound system for 3D imaging as commanded by the macros. A macro, as the term is used herein, comprises a single instruction that expands automatically into a set of instructions that perform a specific task. For example, a particular macro set can, when given a particular user input or a particular reference image orientation, acquire a 3D image data set in relation to the reference image, and form one or more MPR images from the 3D image data which are orthogonal to the orientation of the reference image. The macros can also turn on the user controls necessary to manipulate the 3D images. The sequence of macros produced by the macro storage/processor 42 is coupled to the system controller 12, which causes the instructions of image acquisition macros to be carried out by the beamformer controller 18 to acquire desired image data in relation to the reference image. The system controller causes image formation macros to be carried out by the volume renderer and scan converter for the formation of desired volume and planar images. The macros are also applied to a 3D display processor 36 to direct it to process 3D data sets from the volume renderer 34 into desired 3D images such as MPR images. For instance, a particular set of macros can cause the beamformer controller 18 to command acquisition of a 3D data set in relation to a reference image plane, the volume renderer 34 to render a volume image as viewed from a particular look direction, and the 3D display processor to form three orthogonal MPR images in relation to the center of the volume image. Such a set of macro instructions would cause the ultrasound system to produce the four images shown in the lower-left of the Display area of the user interface 100 of FIGURE 5, for example. The 3D display processor 36 is also responsive to information about the 2D reference image to generate the images and graphics shown in the Display and Controls areas 106 and 108 of the touchscreen user interface 100, including the thumbnails of the display option images and the initial control settings.

A typical set of macro instructions assembled by the macro storage/processor 42 for a reference image which is a long-axis view of the carotid artery is:

**Table 1**

| | |
|---|---|
| 1. | Select display format as "2-up" |
| 2. | Select the images to be displayed as "Volume and A-plane" |
| 3. | Make "volume" image the active image |
| 4 . | Set "Vol" as the active control |
| 5. | Set trackball arbitration to "rotate volume" |
| 6. | Set all 3 rotate cursors as active |
| 7. | Detect circle in B-plane in MPRs and record diameter of detected circle |
| 8. | Place ROI box along center line of detected circle in B-plane |
| 9. | Select ROI box size as 1.2 (diameter of detected circle in step 7) |
| 10. | Select look direction as "top" |
| 11. | Set A-, B-, and C-plane rotate values to 0. |

This sequence of macros will carry out the following actions. The display format will be set for the display of two images ("2-up"). The images will be a 3D (volume) and a 2D planar (A-plane) image in the "A" orientation. The volume image will be a live image and the controls will operate to manipulate the volume image. The volume controls are listed in the Controls area of the user interface touchpanel display. When the user manipulates the trackball, it will cause the volume to rotate in the direction of the trackball motion. Cursors in the image which can be manipulated to rotate the volume about specific axes will all be operational and listed in the Controls area of the user interface. A circle will be detected in each MPR slice image and its diameter will be recorded. An ROI box will be shown on the center line of each circle, and its size set to 1.2cm. The volume image will be initially viewed from the top, and the rotation of three orthogonal planes through the volume is set to zero. As is seen by the above, this set of macros not only commands the acquisition and formation of specific images, but also lists and activates user controls needed for manipulating and measuring them.

Another example of a set of macros which may be assembled by the macro storage/processor 42 is the following:

**Table 2**

| | |
|---|---|
| 1. | Use the 2D reference plane as the start acquisition plane |
| 2. | Use display type as 1-up and acquisition plane as the ROI cut-plane |
| 3. | Set look direction to Top view |
| 4. | Set rotate values to (0,90,0) for A, B, and C planes |
| 5. | Set trackball arbitration to "volume slice" |

This sequence of macros will cause the 2D reference plane to be used as the starting plane for 3D data acquisition. A single image will be displayed and the plane in which image data is acquired is set as a cut-plane through a region of interest and a 3D image is initially viewed from above. Of the three orthogonal planes through a 3D image, only the B plane is rotated, and by 90°. When the trackball is manipulated it will cause the cut-plane through the 3D image to change position.

It is seen from the foregoing that operation of an ultrasound system in the 3D mode is made much simpler for those who are unfamiliar with 3D ultrasound. A user can apply her expertise in standard 2D imaging to acquire a reference image, which is used by the system as the starting point for automatically setting up the system for 3D operation. The user informs the system of the characteristics of the reference image and the system sets up 3D operation for the desired exam type. The system can not only command the acquisition and formation of the appropriate 3D images for the exam, but can also initialize controls and measurement tools needed for the manipulation and assessment of the 3D images.

It should be noted that an ultrasound system suitable for use in an implementation of the present invention, and in particular the component structure of the ultrasound system described in FIGURE 1, may be implemented in hardware, software or a combination thereof. The various embodiments and/or components of an ultrasound system, for example, the modules, or components and controllers therein, also may be implemented as part of one or more computers or processors. The computer or processor may include a microprocessor. The microprocessor may be connected to a communication bus, for example, to access a PACS system or a data network. The computer or processor may also include a memory. The memory devices may include Random Access Memory (RAM) and Read Only Memory (ROM) or other digital or analog signal storage components. The computer or processor further may include a storage device, which may be a hard disk drive or a removable storage drive such as a floppy disk drive, optical disk drive, solid-state thumb drive, and the like. The storage device may also be other similar means for loading computer programs or other instructions into the computer or processor.

As used herein, the term "computer" or "module" or "processor" or "workstation" may include any processor-based or microprocessor-based system including systems using microcontrollers, reduced instruction set computers (RISC), ASICs, logic circuits, and any other circuit or processor capable of executing the functions described herein. The above examples are exemplary only, and are thus not intended to limit in any way the definition and/or meaning of these terms.

The computer or processor executes a set of instructions that are stored in one or more storage elements, in order to process input data. The storage elements may also store data or other information as desired or needed. The storage element may be in the form of an information source or a physical memory element within a processing machine. For example, the macro storage/processor described above comprises a digital memory device which stores digital macro instructions, and a processor that executes instructions which select the appropriate macros which, when executed, set up the ultrasound system for 3D imaging in accordance with the characteristics of the 2D reference image.

The set of instructions of an ultrasound system including those controlling the acquisition, processing, and transmission of ultrasound images as described above may include various commands that instruct a computer or processor as a processing machine to perform specific operations such as the methods and processes of the various embodiments of the invention. The set of instructions may be in the form of a software program. For instance, the ultrasound system of FIGURE 9 may be programmed with instructions executing an algorithm which selects macro instructions from storage that are executed to set up the system for a desired exam type with 3D imaging. The software may be in various forms such as system software or application software and which may be embodied as a tangible and non-transitory computer readable medium. Further, the software may be in the form of a collection of separate programs or modules, a program module within a larger program or a portion of a program module. The software also may include modular programming in the form of object-oriented programming. The processing of input data by the processing machine may be in response to operator commands, or in response to results of previous processing, or in response to a request made by another processing machine.

## Claims

1. An ultrasound system for an exam using 3D imaging, comprising:
a transducer array (10) configured to transmit ultrasound waves to a target anatomy and receive ultrasonic echoes in response;
a processor configured to, based on the received ultrasonic echoes, produce a 2D ultrasound image (110) including the target anatomy; and
a display (38) adapted to display graphical icons,
**characterized in that** the processor is further configured to:
- based on a system input, select a plurality of graphical icons (111, 113, 115, 117, 119) stored in memory on the ultrasound system and cause the display (38) to display said plurality of graphical icons, wherein each graphical icon comprises a different 3D thumbnail view of the target anatomy, and
- based on a user selection of a graphical icon of said plurality, cause the ultrasound system to generate a 3D ultrasound image of the target anatomy corresponding to the thumbnail view of the selected graphical icon.

2. The ultrasound system of Claim 1, wherein the system input comprises a user input that indicates orientation information of the target anatomy with respect to the transducer array (10).

3. The ultrasound system of Claim 2, wherein the user input comprises a text-based or touch-based input configured to identify a viewing orientation of the target anatomy or protocol configured to view the target anatomy.

4. The ultrasound system of Claim 2, wherein the 2D ultrasound image (110) is a reference image, and the user input comprises a selection of the reference image displayed on the display (38), wherein the reference image indicates orientation information of the target anatomy with respect to the transducer array (10).

5. The ultrasound system of Claim 1, wherein the system input comprises orientation information of the target anatomy with respect to the transducer array (10) generated by a segmentation model of the target anatomy.

6. The ultrasound system of Claim 1, further comprising a volume renderer (34) adapted to produce at least one 3D image of the target anatomy that corresponds to at least one of the different 3D thumbnail views in the graphical icons (111, 113, 115, 117, 119).

7. The ultrasound system of Claim 2, further configured to generate a multiplanar view of the target from 3D image data used to produce the at least one 3D image.

8. The ultrasound system of Claim 1, further comprising a system controller (12) that, based on the system input, is adapted to modify 3D image and control settings of the system with a 3D display processor (36).

9. The ultrasound system of Claim 8, wherein the system controller (12) is adapted to modify 3D image and control settings of the system according to a macro stored in memory.

10. The ultrasound system of Claim 9, wherein the system controller (12) is further coupled to receive macros from a macro storage and controller (42), and outputs coupled to an image processor (30) and a volume renderer (34).

11. The ultrasound system of Claim 10, further comprising:
a beamformer (20) having an input coupled to receive signals from the transducer array (10) and an output coupled to the processor; and
a beamformer controller (18) having an output coupled to the beamformer (20),
wherein an output of the system controller (12) is further coupled to the beamformer controller (18).

12. The ultrasound system of Claim 11, wherein the system controller (12) is further adapted to control the beamformer (20), the image processor (30), and the volume renderer (34) in response to its receipt of the macro.

13. The ultrasound system of Claim 1, wherein the image processor(30) further comprises a B mode processor (26) and a Doppler processor (28).

14. The ultrasound system of Claim 1, further comprising a user interface (112) adapted to launch a 3D imaging mode in response to actuation of a user control (116).

15. The ultrasound system of Claim 4, further comprising a user interface (100) having an area (104) adapted to contain characteristics of the reference image.

## Patentansprüche

1. Ultraschallsystem für eine Untersuchung unter Verwendung von 3D-Bildgebung, umfassend:
eine Wandleranordnung (10), die konfiguriert ist, um Ultraschallwellen an eine Zielanatomie zu senden, und als Antwort Ultraschallechos zu empfangen;
einen Prozessor, der konfiguriert ist, um basierend auf den empfangenen Ultraschallechos ein 2D-Ultraschallbild (110) einschließlich der Zielanatomie zu erzeugen; und
eine Anzeige (38) zur Anzeige grafischer Symbole,
**dadurch gekennzeichnet, dass** der Prozessor weiter konfiguriert ist zum:
- basierend auf einer Systemeingabe, Auswählen mehrerer grafischer Symbole (111, 113, 115, 117, 119), die im Speicher des Ultraschallsystems gespeichert sind, und Bewirken, dass die Anzeige (38) die Vielzahl von grafischen Symbolen anzeigt, wobei jedes grafische Symbol eine andere 3D-Miniaturansicht der Zielanatomie umfasst, und
- basierend auf einer Benutzerauswahl eines grafischen Symbols der Vielzahl, Bewirken, dass das Ultraschallsystem ein 3D-Ultraschallbild der Zielanatomie erzeugt, das der Miniaturansicht des ausgewählten grafischen Symbols entspricht.

2. Ultraschallsystem nach Anspruch 1, wobei die Systemeingabe eine Benutzereingabe umfasst, die Orientierungsinformationen der Zielanatomie in Bezug auf die Wandleranordnung (10) anzeigt.

3. Ultraschallsystem nach Anspruch 2, wobei die Benutzereingabe eine textbasierte oder berührungsbasierte Eingabe umfasst, die konfiguriert ist, um eine Betrachtungsorientierung der Zielanatomie oder eines Protokolls zu identifizieren, das konfiguriert ist, um die Zielanatomie anzuzeigen.

4. Ultraschallsystem nach Anspruch 2, wobei das 2D-Ultraschallbild (110) ein Referenzbild ist, und die Benutzereingabe eine Auswahl des auf der Anzeige (38) angezeigten Referenzbildes umfasst, wobei das Referenzbild Orientierungsinformationen der Zielanatomie in Bezug auf die Wandleranordnung (10) anzeigt.

5. Ultraschallsystem nach Anspruch 1, wobei die Systemeingabe Orientierungsinformationen der Zielanatomie in Bezug auf die Wandleranordnung (10) umfasst, die durch ein Segmentierungsmodell der Zielanatomie erzeugt wird.

6. Ultraschallsystem nach Anspruch 1, ferner umfassend eine Volumendarstellung (34), die angepasst ist, um mindestens ein 3D-Bild der Zielanatomie zu erzeugen, das mindestens einer der verschiedenen 3D-Miniaturansichten in den grafischen Symbolen (111, 113, 115, 117, 119) entspricht.

7. Ultraschallsystem nach Anspruch 2, ferner konfiguriert, um eine multiplanare Ansicht des Ziels aus 3D-Bilddaten zu erzeugen, die zur Erzeugung des mindestens einen 3D-Bildes verwendet werden.

8. Ultraschallsystem nach Anspruch 1, ferner umfassend eine Systemsteuerung (12), die basierend auf der Systemeingabe angepasst ist, um das 3D-Bild zu ändern, und die Einstellungen des Systems mit einem 3D-Anzeigeprozessor (36) zu steuern.

9. Ultraschallsystem nach Anspruch 8, wobei die Systemsteuerung (12) angepasst ist, um das 3D-Bild zu ändern, und die Einstellungen des Systems gemäß einem im Speicher gespeicherten Makro zu steuern.

10. Ultraschallsystem nach Anspruch 9, wobei die Systemsteuerung (12) weiter gekoppelt ist, um Makros von einem Makro-Speicher/Controller (42) zu empfangen, und Ausgänge, die an einen Bildprozessor (30) und eine Volumendarstellung (34) gekoppelt sind.

11. Ultraschallsystem nach Anspruch 10, ferner umfassend:
einen Strahlformer (20) mit einem Eingang, der zum Empfangen von Signalen von der Wandleranordnung (10) gekoppelt ist, und einem Ausgang, der mit dem Prozessor gekoppelt ist; und
eine Strahlformersteuerung (18) mit einem Ausgang, der mit dem Strahlformer (20) gekoppelt ist,
wobei ein Ausgang der Systemsteuerung (12) weiter mit der Strahlformersteuerung (18) gekoppelt ist.

12. Ultraschallsystem nach Anspruch 11, wobei die Systemsteuerung (12) ferner angepasst ist, um den Strahlformer (20), den Bildprozessor (30) und die Volumendarstellung (34) als Reaktion auf seinen Empfang des Makros zu steuern.

13. Ultraschallsystem nach Anspruch 1, wobei der Bildprozessor (30) ferner einen B-Modus-Prozessor (26) und einen Doppler-Prozessor (28) umfasst.

14. Ultraschallsystem nach Anspruch 1, ferner umfassend eine Benutzerschnittstelle (112), die zum Starten eines 3D-Bildgebungsmodus als Reaktion auf die Betätigung einer Benutzersteuerung (116) angepasst ist.

15. Ultraschallsystem nach Anspruch 4, ferner umfassend eine Benutzerschnittstelle (100) mit einem Bereich (104), der angepasst ist, um Eigenschaften des Referenzbildes zu enthalten.

## Revendications

1. Système à ultrasons pour un examen utilisant l'imagerie 3D, comprenant:
un réseau de transducteurs (10) configuré pour transmettre des ondes ultrasonores vers une anatomie cible et recevoir des échos ultrasoniques en réponse;
un processeur configuré pour, sur la base des échos ultrasoniques reçus, produire une image ultrasonore 2D (110) comprenant l'anatomie cible; et
un afficheur (38) adapté pour afficher des icônes graphiques,
**caractérisé en ce que** le processeur est en outre configuré pour:
- sur la base d'une entrée de système, sélectionner une pluralité d'icônes graphiques (111, 113, 115, 117, 119) stockée en mémoire sur le système à ultrasons et amener l'afficheur (38) à afficher ladite pluralité d'icônes graphiques, où chaque icône graphique comprend une vue miniature 3D différente de l'anatomie cible, et
- sur la base d'une sélection de l'utilisateur d'une icône graphique parmi ladite pluralité, amener le système à ultrasons à générer une image ultrasonore 3D de l'anatomie cible correspondant à la vue miniature de l'icône graphique sélectionnée.

2. Système à ultrasons selon la revendication 1, dans lequel l'entrée du système comprend une entrée utilisateur qui indique des informations d'orientation de l'anatomie cible par rapport au réseau de transducteurs (10).

3. Système à ultrasons selon la revendication 2, dans lequel l'entrée utilisateur comprend une entrée sous forme de texte ou tactile configurée pour identifier une orientation de visualisation de l'anatomie cible ou un protocole configuré pour visualiser l'anatomie cible.

4. Système à ultrasons selon la revendication 2, dans lequel l'image ultrasonore 2D (110) est une image de référence, et l'entrée utilisateur comprend une sélection de l'image de référence affichée sur l'afficheur (38), où l'image de référence indique des informations d'orientation de l'anatomie cible par rapport au réseau de transducteurs (10).

5. Système à ultrasons selon la revendication 1, dans lequel l'entrée du système comprend des informations d'orientation de l'anatomie cible par rapport au réseau de transducteurs (10) générées par un modèle de segmentation de l'anatomie cible.

6. Système à ultrasons selon la revendication 1, comprenant en outre un rendu volumique (34) adapté pour produire au moins une image 3D de l'anatomie cible qui correspond à au moins l'une des différentes vues miniatures 3D dans les icônes graphiques (111, 113, 115, 117, 119).

7. Système à ultrasons selon la revendication 2, configuré en outre pour générer une vision multiplanaire de la cible à partir de données d'image 3D utilisées pour produire l'au moins une image 3D.

8. Système à ultrasons selon la revendication 1, comprenant en outre un dispositif de commande du système (12) qui, sur la base de l'entrée du système, est adapté pour modifier une image 3D et commander des réglages du système avec un processeur d'affichage 3D (36) .

9. Système à ultrasons selon la revendication 8, dans lequel le dispositif de commande du système (12) est adapté pour modifier une image 3D et commander des réglages du système selon une macro stockée en mémoire.

10. Système à ultrasons selon la revendication 9, dans lequel le dispositif de commande du système (12) est en outre couplé pour recevoir des macros à partir d'un stockage et dispositif de commande de macros (42), et de sorties couplées à un processeur d'image (30) et un rendu volumique (34).

11. Système à ultrasons selon la revendication 10, comprenant en outre:
un formateur de faisceau (20) ayant une entrée couplée pour recevoir des signaux du réseau de transducteurs (10) et une sortie couplée au processeur; et
un dispositif de commande du formateur de faisceau (18) ayant une sortie couplée au formateur de faisceau (20),
où une sortie du dispositif de commande du système (12) est en outre couplée au dispositif de commande du formateur de faisceau (18).

12. Système à ultrasons selon la revendication 11, dans lequel le dispositif de commande du système (12) est en outre adapté pour commander le formateur de faisceau (20), le processeur d'image (30), et le rendu volumique (34) en réponse à sa réception de la macro.

13. Système à ultrasons selon la revendication 1, dans lequel le processeur d'image (30) comprend en outre un processeur mode B (26) et un processeur Doppler (28).

14. Système à ultrasons selon la revendication 1, comprenant en outre une interface utilisateur (112) adaptée pour lancer un mode d'imagerie 3D en réponse à l'actionnement d'une commande d'utilisateur (116).

15. Système à ultrasons selon la revendication 4, comprenant en outre une interface utilisateur (100) ayant une zone (104) adaptée pour contenir des caractéristiques de l'image de référence.
